Europäisches Patentamt

European Patent Office

Office européen des brevets

(19)

(11) Veröffentlichungsnummer: **0 075 878**
**A1**

(12) # EUROPÄISCHE PATENTANMELDUNG

(21) Anmeldenummer: 82108829.1

(22) Anmeldetag: 24.09.82

(51) Int. Cl.³: **A 41 B 13/02**

(30) Priorität: 28.09.81 DE 3138526

(43) Veröffentlichungstag der Anmeldung:
06.04.83 Patentblatt 83/14

(84) Benannte Vertragsstaaten:
AT BE FR GB IT LU NL

(71) Anmelder: Paul Hartmann Aktiengesellschaft
Paul-Hartmann-Strasse
D-7920 Heidenheim(DE)

(72) Erfinder: Eschler, Dieter
Hellensteinstrasse 27
D-7920 Heidenheim(DE)

(74) Vertreter: Becker, Maria, Dipl.-Phys.
Auf dem Haigst 29
D-7000 Stuttgart 70(DE)

(54) **Windelverschluss für eine Wegwerfwindel.**

(57) Die Erfindung betrifft einen Windelverschluß (18) für eine Wegwerfwindel (10). Dieser weist einen ersten und einen zweiten an seiner einen Seite eine Klebeschicht (28 bzw. 30) tragenden streifenförmigen Verschlußteil (20, 22) auf. Der zweite Verschlußteil (22) ist an seiner der Klebeschicht (30) gegenüberliegenden Seite nichtklebend ausgebildet. Mit seiner Klebeschicht (30) ist er im Abstand vom einen Stirnende des ersten Verschlußteils (20) an dessen die Klebeschicht (28) aufweisenden Seite durch Kleben gehalten. Ein Teilstück (26) des ersten Verschlußteils (20) bildet dabei ein Befestigungsteil zu dessen Befestigung an einem anderen Teil der Wegwerfwindel (10).

Zur gegenseitigen Verbindung der beiden Verschlußteile (20, 22) dient ein Zwischenverbindungsglied (42), das an der Klebeschicht (28) des ersten Verschlußteils (20) haftet und an dem das eine Stirnende des zweiten Verschlußteils (22) mit seiner Klebeschicht (30) angeklebt ist. Durch die gegenseitige Verbindung der Verschlußteile (20, 22) mittels des Zwischenverbindungsgliedes (42) werden ausreichende Verbindungskräfte erzielt, wobei die Länge des zweiten Verschlußteils (22) auf die Dimension reduziert werden konnte, entlang welcher das Verschlußteil mit der Windel gänzlich zu verkleben ist. Es läßt sich somit hochwertiges Streifenmaterial einsparen.

Fig. 5

Paul Hartmann AG
D-7920 Heidenheim

# Windelverschluss für eine Wegwerfwindel

Die Erfindung betrifft einen Windelverschluss für eine Wegwerfwindel, bestehend aus mindestens einem ersten und einem zweiten streifenförmigen, an ihrer einen Seite eine Klebeschicht tragenden Verschlussteil, von denen das zweite, an seiner der Klebeschicht gegenüberliegenden Seite, nicht klebend ist und mit seiner Klebeschicht im Abstand vom einen Stirnende des ersten Verschlussteils an dessen die Klebeschicht aufweisenden Seite angeklebt ist, wobei das eine Teilstück des ersten Verschlussteils, das sich von der Verbindungsstelle des zweiten Verschlussteils bis zum einen Stirnende des ersten Verschlussteils erstreckt, ein Befestigungsteil zur Befestigung des ersten Verschlussteils an einem anderen Teil der Windel bildet.

Wegwerfwindeln werden aus übereinander liegenden Materialbahnen hergestellt, wobei die untere Materialbahn eine dünne, bieg- und dehnbare, flüssigkeitsundurchlässige Schicht bildet, auf die eine weiteree, verhältnismässig dicke Schicht aus saugfähigem Material aufgebracht ist, die von einer durchlässigen Deckschicht in Form eines Vlieses bedeckt ist.

Um nun solche Wegwerfwindeln Babies und Kleinkindern in Art einer Hose anlegen zu können, werden im Bereich des einen Stirnendes der sandwichartigen Windelzuschnitte an den einander gegenüberliegenden Längskanten derselben zwei Windelverschlüsse der vorstehend beschriebenen Art angebracht, wobei das eine streifenförmigw Verschlussteil mit seiner Klebeschicht an der dünnen, flüssigkeitsundurchlässigen rückwärtigen Schicht, und das andere Verschlusteil mit seiner Klebeschicht an der oberen Windelseite festgeklebt wird, die mindestens zum grössten Teil durch ein nach oben umgelegtes Randstück der flüssigkeitsundurchlässigen Schicht gebildet ist, welches mit der Deckschicht verbunden ist.

Diese Art der Anordnung der Windelverschlüsse an den Längsrändern von Wegwerfwindeln verteilt am Befestigungsteil wirksam werdende Zugkräfte angelegter Wegwerfwindeln derart, dass diese ungefähr gleichmässig auf diejenigen mit den Verschlussteilen verbundenen Windelteile übertragen werden.

Bei dem bekannten Windelverschluss der erläuterten Art ist die feste Verbindung des zweiten streifenförmigen Verschlussteiles mit dem ersten Verschlussteil in der Weise bewerkstelligt, dass dessen eines Stirnende über die gesamte Breite des Verschlussteils mit dem anderen Verschlussteil verklebt ist, wobei der Klebebereich, in der Länge dieses Verschlussteils gesehen, 2 bis 3 mm beträgt.

Bei den zur Verwendung kommenden Verschlussteilen handelt es sich um hochwertiges Streifenmaterial, das in der Lage ist, den bei angelegter Wegwerfwindel durch dynamische Kräfte ausgelösten hohen Zugkräften eine entsprechend hohe Zugfestigkeit entgegenzusetzen. Dabei ist das zweite Verbindungsteil, das auf seiner der Klebeschicht gegenüberliegenden Seite eine sogenannte Ablöseschicht besitzen muss, die keine Klebeverbindung mit einer Klebeschicht eingeht, aus besonders teurem Material zu fertigen.

Der Nachteil dieses bekannten Windelverschlusses ist deshalb sofort erkennbar, wenn man berücksichtigt, dass 2 bis 3 mm der Gesamtlänge dieses teuren Verschlussteils zur Herstellung einer Haftverbindung mit dem anderen Verschlussteil benötigt werden.

Bei dieser Länge und der gegebenen Breite der beiden gleich breiten Verschlussteile ergibt sich damit ein erheblicher Flächenanteil, der am zweiten Verschlussteil zwar benötigt wird, jedoch für die herzustellende Klebeverbindung des Windelverschlusses mit der Wegwerfwindel nicht mehr zur Verfügung steht.

Der vorliegenden Erfindung liegt von da her die Aufgabe zugrunde, bei Windelverschlüssen dieser bekannten Art den Materialanteil des besonders teuren zweiten Verschlussteils zu senken bzw. eine günstigere Art der gegenseitigen Verbindung der beiden Verschlussteile miteinander zu schaffen. Diese Aufgabe wird erfindungsgemäss dadurch gelöst, dass zur gegenseitigen Verbindung der beiden Verschlussteile aus dem einen streifenförmigen Verschlussteil mindestens eine Anhängezunge ausgestanzt ist, die entlang einer quer zur Längsrichtung dieses Verschlussteils verlaufenden Biegekante mit diesem verbunden ist, und dass an der die Klebeschicht tragenden Zungenseite das andere Verschlussteil mit seiner dessen Klebeschicht aufweisenden Seite angeklebt ist.

Die Erfindung basiert auf der Erkenntnis, dass die erläuterte Klebeverbindung der beiden Verschlussteile des bekannten Windelverschlusses überdimensioniert ist, was jedoch aus fertigungstechnischen Gründen in Kauf genommen wurde.

Demgegenüber konnte durch die Erfindung eine Verbindung geschaffen werden, die bei weitem ausreichende Verbindungskräfte erzielt, dabei jedoch den besonderen Vorteil ermöglicht, die Länge des zweiten Verschlussteils auf die Dimension zu reduzieren, entlang welcher das Verschlussteil mit der Windel gänzlich zu verkleben ist. Die Erfindung ermöglicht es damit, besonders hochwertiges Streifenmaterial je Windelverschluss in erheblichem Umfang einzusparen.

Die Erfindung erfordert zwar eine Stanzoperation, jedoch sind die hierfür anzusetzenden Kosten geringer als diejenigen, die bei den bekannten Windelverschlüssen für die verlorene Überlänge des zweiten Verschlussteils aufzuwenden sind.

Bevorzugt wird eine Ausbildung des Windelverschlusses, bei der die Anhängezunge aus dem ersten Verschlussteil herausgestanzt ist, so dass das an die Anhängezunge angeklebte zweite Verschlussteil mit seiner Ablöseschicht an der Klebeschicht des anderen Verschlussteils ablösbar angelegt werden kann.

Dabei ist es günstig, wenn der Abstand der Biegekante der Anhängezunge vom einen Stirnende des sie tragenden ersten Verschlussteils im wesentlichen der Länge des an ihr haftenden anderen Verschlussteils entspricht.

Die insbesondere länglich ausgebildete Anhängezunge kann vorteilhaft in der Quermitte des sie tragenden Verschlussteils angeordnet sein. Es können aber auch mehrere, beispielsweise zwei Anhängezungen vorgesehen sein, die im Bereich der Längsränder des sie tragenden Verschlussteils vorgesehen sind und deren Biegekanten sich in einer gemeinsamen Ebene befinden.

Selbständiger Gegenstand der Erfindung ist ein Windelverschluss, bestehend aus mindestens einem ersten und einem zweiten streifenförmigen Verschlussteil, entsprechend der eingangs aufgeführten Ausbildung, bei dem das zweite Verschlussteil an seiner der Klebeschicht gegenüberliegenden Seite nicht klebend ausgebildet ist und mit seiner Klebeschicht im Abstand vom einen Stirnende des ersten Verschlussteils an dessen die Klebeschicht aufweisenden Seite gehalten ist. Dieser Windelverschluss zeichnet sich erfindungsgemäss dadurch aus, dass zur gegenseitigen Verbindung der beiden Verschlussteile ein Zwischenverbindungsglied dient, das an der Klebeschicht des ersten Verschlussteils haftet und an dem das eine Stirnende des zweiten Verschlussteils mit seiner Klebeschicht angeklebt ist.

Dieser Windelverschluss ermöglicht ebenfalls eine vorteilhafte Einleitung von am Befestigungsteil wirksamen Zugkräften in einander gegenüberliegende Schichten der Windel im Bereich einer Längskante derselben.

Besonders vorteilhaft eignet sich hierbei eine Ausführungsform, bei der das Zwischenverbindungsglied in an eine Wegwerfwindel angebrachtem Zustand eine Längskante derselben umgreift, so dass eine besonders wirksame Haftverbindung des Windelverschlusses mit der Windel sich dadurch zustandebringen lässt, dass das streifenförmige Zwischenverbindungsglied auf beiden Seiten eine Klebeschicht besitzt.

Weitere Merkmale und Einzelheiten der Erfindung sind in der sich anschliessenden Beschreibung von in der Zeichnung gezeigten Ausführungsbeispielen erfindungsgemässer Windelverschlüsse und/oder in den Ansprüchen erläutert. In der Zeichnung zeigen:

Figur 1 eine schaubildliche Darstellung eines ersten Ausführungsbeispiels eines an einem Längsrand einer Wegwerfwindel befestigten, erfindungsgemässen Windelverschlusses,

Figur 2 eine Vorderansicht des Windelverschlusses,

Figur 2a eine Darstellung ähnlich Figur 2 einer Konstruktionsvariante eines Windelverschlusses,

Figur 3 einen Längsschnitt durch den Windelverschluss gemäss Figuren 1 und 2,

Figur 4 eine schaubildliche Darstellung eines zweiten Ausführungsbeispiels eines an eine Wegwerfwindel angebrachten Windelverschlusses,

Figur 5 einen Längsschnitt des Windelverschlusses entlang der Linie 5-5 der Figur 4.

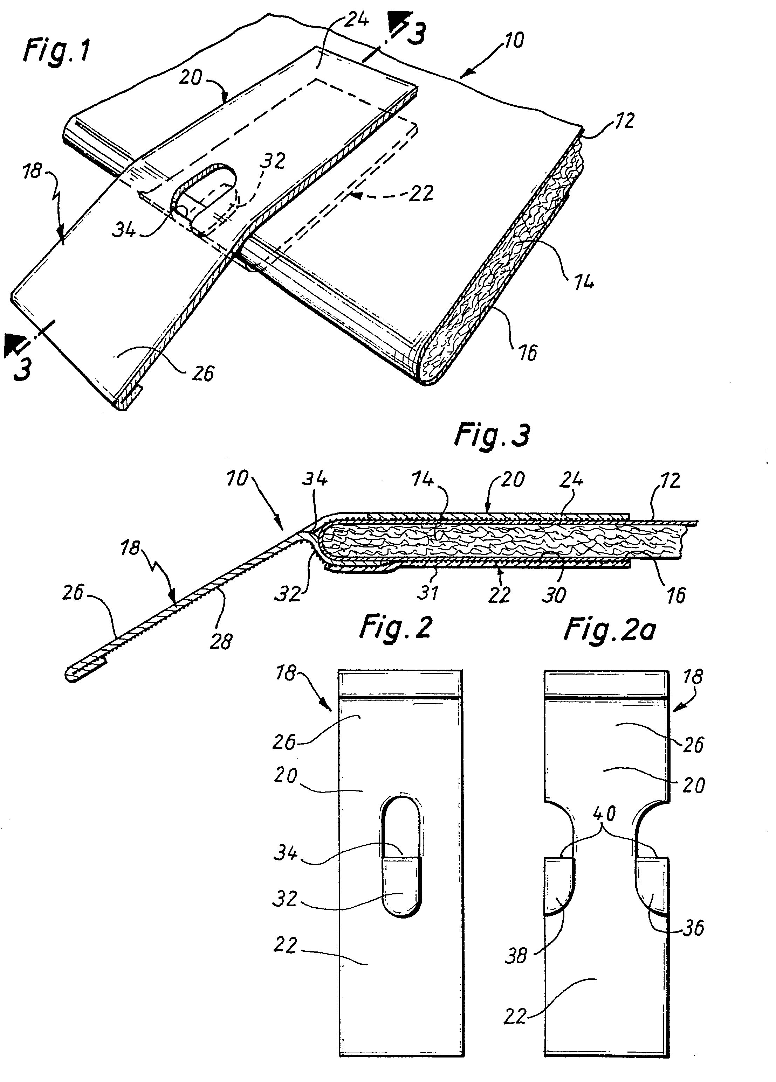

In Figur 1 ist das eine Längsrandstück einer als Ganzes mit 10 bezeichneten Wegwerfwindel dargestellt. Diese ist sandwichartig aufgebaut, wobei mit 12 eine rückwärtige dünne Schicht bezeichnet ist, die biegsam, dehnbar und flüssigkeitsundurchlässig ist. Auf diese Schicht ist eine verhältnismässig dicke, aus saugfähigem Material bestehende Schicht 14 aufgebracht. Die Längsrandteile der dünnen rückwärtigen Schicht 12 sind um die saugfähige Schicht 14 herumgelegt, und die auf dieser obenauf liegenden Längsrandteilstücke der Schicht 12 sind mit 16 bezeichnet.

An beiden Längsrandteilen der Wegwerfwindel sind im Bereich ihres einen Stirnendes an einander gegenüberliegenden Stellen Windelverschlüsse angeordnet, wobei 18 einen derselben bezeichnet. Dieser Windelverschluss besitzt zwei streifenförmige Verschlussteile, die mit 20 und 22 bezeichnet sind.

Das Verschlusteil 20 ist streifenförmig und flexibel ausgebildet, wobei ein Teilstück desselben ein Verbindungsteil 24 bildet, das mit der rückwärtigen Schicht 12 fest verbunden ist und an das sich ein Befestigungsteil 26 anschliesst, das sich über den Längsrand der Wegwerfwindel hinaus erstreckt. Dieses erste Verschlusteil 20 besitzt hierbei an seiner einen Seite durchgehend eine Klebeschicht 28.

Das zweite Verschlusteil 22 haftet am oberen Längsrandteilstück 16 der rückwärtigen Schicht 12, und zwar vorzugsweise deckungsgleich zum Verbindungsteil 24 des ersten Verschlusteils. Die Länge des Verschlusteils 22 kann auch derart gewählt sein, dass es zum Teil auch auf der saugfähigen Schicht 14 haftet. Hierzu ist auch das Verschlusteil 22 an seiner einen Seite mit einer Klebeschicht 30 ausgestattet, während es an seiner gegenüberliegenden Seite eine Ablöseschicht 31 besitzt bzw. nicht klebend ist.

Ausserdem ist dieses Verschlusteil an seinem einen Stirnende fest mit dem Verschlusteil 20 verbunden, wozu aus letzterem eine Anhängezunge 32 ausgestanzt ist, die entlang einer quer zur Längsrichtung des Verschlusteils 20 verlaufenden Biegekante 34 mit diesem verbunden ist. Dabei ist die die Klebeschicht 28 tragende Zungenseite mit der Seite des zweiten Verschlusteils 22 verklebt, welche die Klebeschicht 30 trägt (Figur 3).

Die Anhängezunge 32 ist länglich ausgebildet und befindet sich in der Quermitte des Verschlusteils 20. In der Breite ist die Anhängezunge derart ausgebildet, dass diese ungefähr einem Drittel der Breite des Verschlusteils 20 entspricht. Beim gezeigten Ausführungsbeispiel ist die Anhängezunge länglich ausgebildet. Sie kann ebensogut halbrundförmig oder trapezförmig sein.

Figur 2a zeigt eine mögliche Konstruktionsvariante, bei der beispielsweise zwei Anhängezungen 36, 38 vorgesehen sind, die im Bereich der Längsränder des Verschlussteils 20 vorgesehen sind und deren Biegekanten 40 sich in einer gemeinsamen Querebene befinden. Ebenso könnte die Ausführungsform nach Figur 2a gegebenenfalls auch noch eine mittlere Anhängezunge besitzen, wobei in diesem Falle die Breite der einzelnen Anhängezungen vorzugsweise so gewählt werden sollte, dass Verbindungsteil 24 und Befestigungsteil 26 über Bereiche miteinander verbunden sind, die demjenigen der Gesamtbreite der Anhängezungen entsprechen.

Die aus Figur 3 ersichtliche Anordnung des Windelverschlusses lässt erkennen, dass an dem an einem anderen Windelteil mit der Klebeschicht 28 befestigbaren Befestigungsteil 26 wirksam werdende Zugkräfte einmal direkt in das an der rückwärtigen Schicht 12 anhaftende Verbindungsteil 24 des Verschlussteils 20 und zum anderen über die Anhängezunge 32 auch in das zweite, am Längsrandteilstück 16 haftende zweite Verschlussteil 22 eingeleitet werden, was bedeutet, dass an der Innen- und Aussenseite des betreffenden Windellängsrandes die Zugkräfte im wesentlichen gleich stark wirksam werden und dadurch gewährleistet ist, dass auch bei verhältnismässig grossen Zugkräften eine einwandfreie Halterung des Windelverschlusses an der Wegwerfwindel besteht.

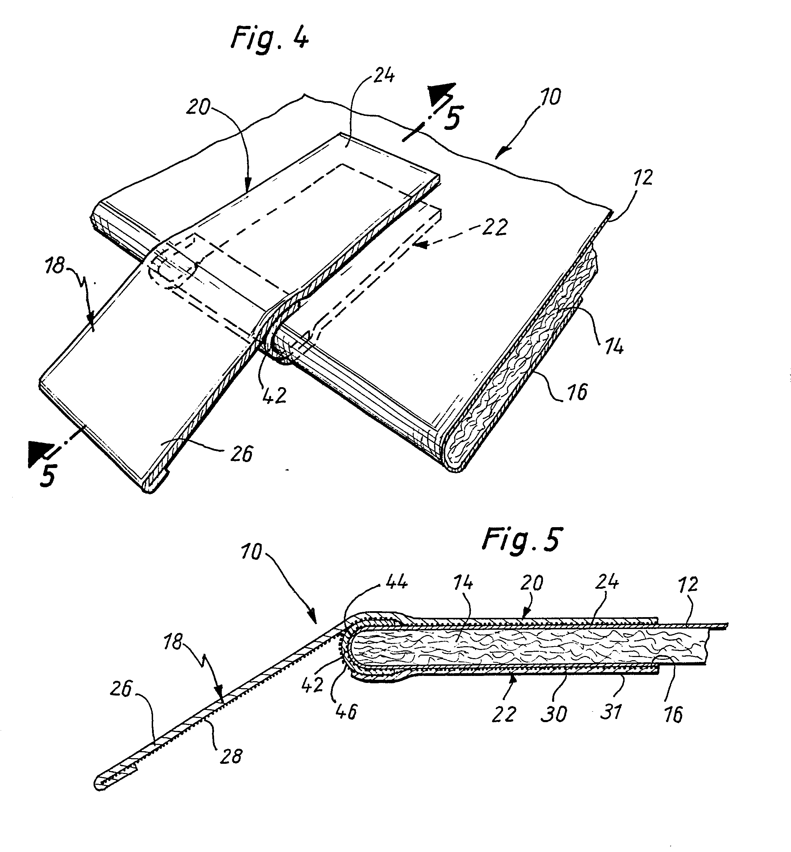

Bei der in den Figuren 4 und 5 gezeigten Ausführungsform eines Windelverschlusses sind diejenigen Teile, die mit den Teilen des vorbeschriebenen Windelverschlusses übereinstimmen, mit gleichen Bezugszahlen bezeichnet. Der Unterschied in der Ausbildung beider Windelverschlüsse zueinander besteht darin, dass zur gegenseitigen Verbindung der beiden Verschlussteile 20 und 22 ein Zwischenverbindungsglied 42 in Form eines auf beiden Seiten mit einer Klebeschicht 44, 46 ausgestatteten Klebebandes vorgesehen ist, das sich quer über das Verschlussteil 20 erstreckt. Die Anordnung des Verschlussteils 20 am Zwischenverbindungsglied 42 ist hierbei so getroffen, dass dessen Klebeschicht 30 und die Klebeschicht 46 des Zwischenverbindungsgliedes 42 aufeinander liegen.

Aus der Darstellung gemäss Figur 5 ist ersichtlich, dass der an einen Längsrandteil einer Wegwerfwindel angebrachte Windelverschluss mit seinem im Querschnitt U-förmigen Verbindungsglied die Längskante der Windel umgreift und mit dessen Klebeschicht 44 an dieser haftet.

Eine weitere selbständig schutzwürdige, jedoch nicht gezeichnete Konstruktion als Variante zur Ausführungsform gemäss den Figuren 4 und 5 zeichnet sich durch lediglich zwei Teile, nämlich das streifenförmige Verschlussteil 20 und das streifenförmige Verschlussteil 22, aus. Das streifenförmige Verschlussteil 22 ist dabei im Bereich seines mit dem Verschlussteil 20 verbundenen Endstückes an beiden Seiten mit einer Klebeschicht ausgestattet, so dass es mit diesem Endstück unter Umgehung eines Zwischenverbindungsgliedes 42 unmittelbar am Verschlussteil 20 durch Kleben gehalten ist. Diese Konstruktion erlaubt durch Wegfall des Zwischenverbindungsgliedes 42 eine erhebliche fertigungstechnische Vereinfachung.

1

Patentansprüche:

1. Windelverschluss für eine Wegwerfwindel, bestehend aus mindestens einem ersten und einem zweiten streifenförmigen, an ihrer einen Seite eine Klebeschicht tragenden Verschlussteil, von denen das zweite, an seiner der Klebeschicht gegenüberliegenden Seite, nicht klebend ist und mit seiner Klebeschicht im Abstand vom einen Stirnende des ersten Verschlussteils an dessen die Klebeschicht aufweisenden Seite angeklebt ist, wobei das eine Teilstück des ersten Verschlussteils, das sich von der Verbindungsstelle des zweiten Verschlussteils bis zum einen Stirnende des ersten Verschlussteils erstreckt, ein Befestigungsteil zur Befestigung des ersten Verschlussteils an einem anderen Teil der Windel bildet,
dadurch gekennzeichnet,
dass zur gegenseitigen Verbindung der beiden Verschlussteile (20, 22) aus dem einen streifenförmigen Verschlussteil (20) mindestens eine Anhängezunge (32) ausgestanzt ist, die entlang einer quer zur Längsrichtung dieses Verschlussteils verlaufenden Biegekante (34) mit diesem verbunden ist, und dass an der die Klebeschicht (28) tragenden Zungenseite das andere Verschlussteil (22) mit seiner dessen Klebeschicht (30) aufweisenden Seite angeklebt ist.

2. Windelverschluss nach Anspruch 1, dadurch gekennzeichnet, dass das an die aus dem ersten Verschlussteil (20) herausgestanzte Anhängezunge (32) angeklebte zweite Verschlussteil (22) mit seiner Ablöseschicht (31) an der Klebeschicht (28) des anderen Verschlussteils (20) ablösbar haftet.

3. Windelverschluss nach Anspruch 2, dadurch gekennzeichnet, dass der Abstand der Biegekante (34) der Anhängezunge (32) vom einen Stirnende des sie tragenden ersten Verschlussteils (20) im wesentlichen der Länge des an ihr haftenden anderen Verschlussteils (22) entspricht.

4. Windelverschluss nach einem der vorhergehenden Ansprüche, dadurch gekennzeichnet, dass die insbesondere länglich ausgebildete Anhängezunge (32) in der Quermitte des sie tragenden ersten Verschlussteils (20) angeordnet ist.

5. Windelverschluss nach einem der vorhergehenden Ansprüche 1 bis 3, gekennzeichnet durch mindestens zwei Anhängezungen (36, 38), die im Bereich der Längsränder des sie tragenden ersten Verschlussteils (20) vorgesehen sind und deren Biegekanten (40) sich in einer gemeinsamen Querebene befinden.

6. Windelverschluss für eine Wegwerfwindel, bestehend aus mindestens einem ersten und einem zweiten streifenförmigen, an ihrer einen Seite eine Klebeschicht tragenden Verschlussteil, von denen das zweite an seiner der Klebeschicht gegenüberliegenden Seite nicht klebend ausgebildet ist und mit seiner Klebeschicht im Abstand vom einen Stirnende des ersten Verschlussteils an dessen die Klebeschicht aufweisenden Seite gehalten ist, wobei das eine Teilstück des ersten Verschlussteils, das sich von der Verbindungsstelle des zweiten Verschlussteils bis zum einen Stirnende des ersten Verschlussteils erstreckt, ein Befestigungsteil zur Befestigung des ersten Verschlussteils an einem anderen Teil der Windel bildet, dadurch gekennzeichnet, dass zur gegenseitigen Verbindung der beiden Verschlussteile (20, 22) ein Zwischenverbindungsglied (42) dient, das an der Klebeschicht (28) des ersten Verschlussteils (20) haftet und an dem das eine Stirnende des zweiten Verschlussteils (22) mit seiner Klebeschicht (30) angeklebt ist.

7. Windelverschluss nach Anspruch 6, dadurch gekennzeichnet, dass das Zwischenverbindungsglied (42) in an eine Wegwerfwindel angebrachtem Zustand eine Längskante derselben umgreift.

8. Wegwerfwindel nach Anspruch 6 oder 7, dadurch gekennzeichnet, dass das streifenförmige Zwischenverbindungsglied (42) auf beiden Seiten eine Klebeschicht (44, 46) besitzt.

0075878

1/2

Fig.1

24

20

3

10

18

32

22

34

12

26

14

16

Fig.3

10

34

14

20

24

12

18

32

31

22

30

16

26

28

Fig.2

Fig.2a

18

18

26

26

20

20

34

40

32

36

22

38

22

Fig. 4

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | A 41 B 13/02 |
| X | US-A-4 002 172 (JOHNSON & JOHNSON) * Spalte 3, Zeilen 12-20; Spalte 4, Zeilen 40-68; Spalten 5,6; Spalte 7, Zeilen 1-3; Figuren * | 1-4 | |
| | --- | | |
| A | US-A-3 967 622 (JOHNSON & JOHNSON) * Spalte 3, Zeilen 57-68; Spalte 4, Zeilen 1-61; Figuren 1-7 * | 5 | |
| | --- | | |
| A | US-A-3 848 594 (THE PROCTER & GAMBLE COMPANY) * Spalte 3, Zeilen 42-68; Spalte 4, Zeilen 1-21, 42-68; Spalte 5, Zeilen 1,2, 26-56; Anspruch 1; Figuren 1,3 * | 6 | |
| | --- | | RECHERCHIERTE SACHGEBIETE (Int. Cl. ³) |
| A | US-A-4 044 767 (JOHNSON & JOHNSON) * Spalte 4, Zeilen 26-68; Spalte 5, Zeilen 1-38; Figuren 1-3 * | 6 | A 41 B |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 27-12-1982 | GARNIER F.M.A.C. |